# EUROPEAN PATENT APPLICATION

(11) **EP 1 426 031 A1**
(43) Date of publication of application: **09.06.2004**
(21) Application number: 02762848.6
(22) Date of filing: 23.08.2002
(51) Int. Cl.: A61K 7/02, A61K 7/00, A61K 7/035, A61K 7/42, A61K 7/48, C09C 3/10, C09C 3/12

(54) **MODIFIED INORGANIC FINE GRAINS FOR COSMETICS AND COSMETICS**

(30) Priority: 24.08.2001 JP 2001254992
(71) Applicant: Catalysts & Chemicals Industries Co., Ltd., Kawasaki-shi, Kanagawa 212-0013 (JP)
(72) Inventor: YAMADA, Kenji, Kitakyushu-shi, Fukuoka 802-0837 (JP); MIYAZAKI, Takumi, Catalysts & Chem. Ind. Co. Ltd, Kitakyushu-shi, Fukuoka 808-0027 (JP); TANAKA, Hirokazu, Catalysts & Chem. Ind. Co. Ltd, Kitakyushu-shi, Fukuoka 808-0027 (JP)
(74) Representative: Westendorp, Michael, Dr.
(86) International application number: PCT/JP2002/008539
(87) International publication number: WO 2003/017953

(57) **Abstract**

It is intended to provide fine particles for cosmetics which are excellent in smoothness upon application to the skin or hydrophilic nature and powdery texture. These modified inorganic fine particles comprise, on the surface of inorganic fine particles, a polymer layer (I) prepared by plasma-polymerizing one of monomers selected from among fluorinated monomers, silicone-based monomers, aromatic monomers and alicyclic monomers; a polymer layer (II) obtained by plasma graft-polymerizing a monomer having a functional group; or a polymer layer (III) prepared by plasma graft-polymerizing a monomer having a functional group modified with a functional organic compound(s). The functional organic compound(s) as described above are one or more members selected from among dyes, UV absorbers and skin-chapping inhibitors.

## Description

### TECHNICAL FIELD

The present invention relates to inorganic fine particles for cosmetics surface-modified by coating a specific polymer layer and to cosmetics blending the inorganic fine particles.

### BACKGROUND OF THE INVENTION

There have been known various methods of surface modification to facilitate functionalization of inorganic fine particles, such as water repellent treatment by silicone treatment, water and oil repellent treatment by fluorine treatment, metallic soap treatment, and treatment with a silane coupling agent. These treatments are comprised of methods of chemically or physically binding a treatment agent with a surface of inorganic particles by impregnating an organic compound as a treatment agent with inorganic particles, or by reacting and then heat-treating the same according to the necessity. This surface modification has an objective of, for example, improving compatibility of inorganic fine particles with components in a solvent, giving water and/or oil repellency to inorganic fine particles, or the like. These methods, however, have the problems that a bond between an organic compound as a treatment agent and inorganic particles is unhomogeneous, or, in case of a physical bond, when inorganic particles are mixed with a solvent medium high in compatibility with a surface modifying agent as a solvent and the mixture is then left untouched for a period of time, the physical bond is released to lose the surface modifying effect or the like.

An example of fixing an organic compound having functionality such as dyes and UV absorbers to inorganic fine particles includes a method of fixing dyes to inorganic fine particles such as pearl pigments. Organic dyes such as acid dyes and organic pigments have a significantly high chroma and a bright color tone, however, when any of these only is used to the skin without fixing them to inorganic fine particles or the like, their sorbability and absorbency to the skin is so high to cause a problem of safety. In order to avoid this problem, when organic dyes or pigments are fixed to inorganic fine particles, there is no way available other than utilizing various chelate formation reactions, so that, for example, the method of forming chelate with various dyes using aluminum hydroxide to fix dyes has been proposed (Japanese Patent No. 1258216). In these methods as described above, however, dyes sometimes break loose under specific conditions, for example, in an acid atmosphere or in an alkali atmosphere, and a complete fixing is difficult.

On the other hand, plasma polymerization and plasma graft polymerization has been known as a dry process in which treatment such as drying or cleaning is not required, for example, the method of plasma-polymerizing or plasma graft-polymerizing various monomers with a resin base in the form of sheet or fiber to surface-modify inorganic fine particles such as giving hydrophilicity, or the method of improving texture of fibers utilizing similar treatments as described above (Japanese Patent Publication No. HEI 5-65618, Japanese Patent Laid-Open Publication No. SHO 61-239083). As a method using inorganic fine particles, the applicants of the present invention has proposed surface-modified silica in which a surface of spherical silica with the average particle size of 20 to 300 µm is coated with polymer layers bonding polymethacrylic acid glycidyl or the like in the form of graft (Japanese Patent Laid-Open Publication No. 2000-143230).

### DISCLOSURE OF THE INVENTION

An object of the present invention is to provide fine particles for cosmetics which are improved in dispersibility for a specific solvent medium and are excellent in smoothness upon application to the skin by modifying a surface of inorganic fine particles, fine particles for cosmetics which are excellent in hydrophilic nature, powdery texture or the like, and fine particles for cosmetics which are excellent in water repellency, oil repellency, colorability, UV blocking performance, and skin-chapping preventing performance (moisture retaining performance). Another object of the present invention is to provide cosmetics blending the fine particles described above.

Modified inorganic fine particles according to the present invention for cosmetics are characterized in that the inorganic fine particles have on a surface thereof, a polymer layer (I) prepared by plasma-polymerizing one or more monomers selected from among fluorinated monomers, silicone-based monomers, aromatic monomers and alicyclic monomers. The weight ratio of the polymer layer (1) to the inorganic fine particles is preferably in the range from 0.1/99.9 to 30/70.

Modified inorganic fine particles for cosmetics according to the present invention are characterized in that the inorganic fine particles have on a surface thereof, a polymer layer (II) obtained by plasma graft-polymerizing a monomer having a functional group. The weight ratio of the polymer layer (II) to the inorganic fine particles is preferably in the range from 0.1/99.9 to 30/70.

Modified inorganic fine particles for cosmetics according to the present invention preferably have a polymer layer (III) with the functional group bonding a functional organic compound(s) therewith. The functional organic compound(s) are preferably one or more members selected from among dyes, UV absorbers and skin-chapping inhibitors. The bonding quantity of the functional organic compound(s) is preferably in the range from 1 to 20% by weight to the weight of the inorganic fine particles forming the polymer layer (II).

Cosmetics according to the present invention is characterized in blending therein 1 to 90% by weight of the modified inorganic fine particles for cosmetics described above.

### BEST MODE FOR CARRYING OUT THE INVENTION

The preferred embodiments of the inorganic fine particles for cosmetics and the cosmetics according to the present invention are described below.

### (Inorganic Fine Particles)

Examples of inorganic fine particles used in the present invention are; clay mineral particles including mica, talc, sericite, synthetic mica, synthetic sericite, barium sulfate, boron nitride, kaolin, and bentonite; plate particles including mica coated with titanium oxide, plate silica, and plate titanium oxide, plate alumina; titanium oxide, silica, alumina, iron oxide, zinc oxide, cerium oxide, magnesium oxide, or the like, or spherical or non-spherical mixed particles or composite particles consisting one or more members thereof; and metallic particles including gold, silver, aluminum, and the like.

When plate particles are used, the average particle size of the inorganic fine particles is in the range from 0.1 to 100 µm, and preferably, from 1 to 50 µm, and the average particle thickness thereof is in the range from 0.02 to 5 µm, and preferably, from 0.1 to 1 µm. When the average particle size of the inorganic fine particles is less than 0.1 µm, coating with a uniform polymer layer is difficult, in the meantime, when the average particle size thereof is more than 100 µm, gloss may sometimes be too strong to use for blending in cosmetics. Further, when the average particle thickness thereof is less than 0.02 µm, the fine particles are easily broken, in the meantime, when the average thickness thereof is more than 5 µm, the fine particles have deteriorated adherability to the skin and are not suitable as ingredients for cosmetics. When the fine particles have other forms, for example, they are spherical particles or non-spherical particles, the average particle size of the fine particles is in the range from 0.05 to 100 µm, and preferably, from 0.2 to 20 µm. When the average particle size thereof is less than 0.05 µm, coating with a uniform polymer layer is also difficult, in the meantime, when the average particle size thereof is more than 100 µm, the fine particles have rough feel and are not suitable as ingredients for cosmetics, either.

### (First Modified Inorganic Fine Particles for Cosmetics)

First modified inorganic fine particles according to the present invention are formed on a surface thereof with a polymer layer (I) prepared by plasma-polymerizing one or more monomers selected from among fluorinated monomers, silicone-based monomers, aromatic monomers and alicyclic monomers.

Fluorinated monomers used in the present invention may include, for example, various fluorine compounds such as hexafluorobenzene, tetrafluoromethane, trifluoromethane, hexafluoroethane, tetrafluoroethane, trifluoroethylene, and vinylidene fluoride.

As silicone-based monomers, ethyl trimethylsilane, vinyl trimethylsilane, dimetyl chlorosilane, vinyl trichlorosilane, vinyl trimethoxysilane, trimethyl silanol, hexametyldisiloxane, trimethylaminosilane or the like may be used.

As aromatic monomers, styrene, benzene, toluene, xylene, or the like, and as alicyclic monomers, cyclohexane, cyclobutane, tetrahydrofuran, or the like may be listed for use in the present invention. Only one of these monomers may be used, or a mixture thereof may also be used.

There is not any particular limit to the method of plasma polymerization and the well-known method in the art may be employed, while the method of low-temperature plasma treatment makes it relatively easy to produce the polymer layer (I), which is preferable.

The method of low-temperature plasma treatment enables plasma irradiation using a commercially available plasma treatment device. Regular plasma treatment conditions are adoptable for the plasma irradiation conditions. For example, inorganic fine particles are put in a rotating container made of pyrex glass or the like, which is then evacuated to displace the inside thereof with monomer gas and to be adjusted to have a prespecified pressure. By applying high-frequency electric power with 13.56 MHz to a copper pipe coil wound around an outer circumference of the container, plasma is generated in the rotating container with the induction system to plasma polymerize monomers on a surface of the inorganic fine particles. Rotational velocity of the container during the plasma treatment is preferably about 20 to 30 rpm. Appropriate plasma treatment conditions are as follows:

| | |
|---|---|
| Discharge gas | Monomer |
| Gas flow rate | (1-40) × 10⁻² dm³/minute |
| Power | 30-200 W |
| Discharge time | 5-30 minutes |
| Pressure | 0.005-100 Torr |

Pressure and power in the container may be selected according to the necessity taking the type and reactivity of inorganic fine particles and monomers into consideration. The afterglow method may also be employed in which prespecified gas such as hydrogen, argon, oxygen or the like is turned into the plasma state in advance, and the monomers are then made to flow through the gas.

The weight of the polymer layer (1) formed by the plasma polymerization described above is preferably in the range from 0.1/99.9 to 30/70, and especially preferably, in the range from 1/99 to 10/90, as the weight ratio to the inorganic fine particles to be coated. When the weight ratio thereof is more than 30/70, the polymer layer (resin layer) is excessive and easily agglomerates upon polymerization, which prevents a uniform polymer layer from being produced. When the weight ratio is less than 0.1/99.9, in turn, sufficient water repellency, oil repellency, or the like may not be obtained.

### (Second Modified Inorganic Fine Particles for Cosmetics)

Second modified inorganic fine particles according to the present invention are formed on a surface thereof with a polymer layer (II) prepared by plasma graft-polymerizing a monomer having a functional group.

Monomers having a functional group used in the present invention may include, acyrilamide, glycidyl methacrylate, acrylic acid, methacrylic acid, N,N-methylene-bisacrylamide, N-isopropyl acrylamide, 2-hydroxydiethyl acrylate, 2-acrylamide-2-methylpropanesulfonic acid, vinylsulfonic acid, and methacrylic sulfonic acid, as well as monomers such as ammonium salt and alkali metallic salt thereof capable of graft-polymerization and having a functional group such as an amide group, a glycidyl group, a carboxylic group, a sulfonic acid group, a hydroxyl group, or the like.

In the present invention, a mixture of two or more of these monomers may be used, or a mixture of one or more of these monomers with other monomers not having a function group may also be used.

There is not any particular limit to the method of plasma graft polymerization and the method proposed in Japanese Patent Laid-Open Publication No. 2000-143230 described above is advantageous. This method employs the technique of low-temperature plasma treatment and makes it relatively easy to produce a polymer layer. Namely, in the method, inorganic fine particles are irradiated by plasma to generate radicals. This plasma irradiation can be carried out using a commercially available plasma treatment device. Any regular plasma treatment conditions are adoptable for the plasma irradiation conditions as far as radicals are generated. More specifically, inorganic fine particles are put in a rotating container made of pyrex glass, which is then evacuated to displace the inside thereof with argon gas and to be adjusted to have a prespecified pressure. By applying high-frequency electric power with 13.56 MHz to a copper pipe coil wound around an outer circumference of the container, plasma is generated in the rotating container with the induction system to generate radicals through irradiating plasma on a surface of the inorganic fine particles. Rotational velocity of the container during the plasma treatment is preferably about 20 to 30 rpm. Appropriate plasma treatment conditions are as follows:

| | |
|---|---|
| Discharge gas | Inert gas |
| Gas flow rate | (1-40) × 10⁻² dm³/minute |
| Power | 50-300 W |
| Discharge time | 1-30 minutes |
| Pressure | 0.005-100 Torr |

The plasma-irradiated inorganic fine particles are transferred into the container with monomers therein without exposing to the atmosphere to contact and polymerize the inorganic fine particles generating radicals with monomers, so that the polymer layer (II) bonding in the form of graft on a surface of the particles is formed. When a monomer is used in the form of liquid solution, the concentration thereof is preferably adjusted in the range from 10 to 50% by volume, and as a solvent medium, a commonly-used organic solvent, water, or a mixture of water and an organic solvent may be used. After the formation of the polymer layer (II), inorganic fine particles forming the polymer layer (II) can be obtained follows cleaning with alcohol or the like and drying of the polymer layer (II).

The weight of the polymer layer (II) coating inorganic fine particles is preferably in the range from 0.1/99.9 to 30/70, and especially preferably, in the range from 1/99 to 10/90, as the weight ratio to the inorganic fine particles. When the weight ratio is more than 30/70, the polymer layer is excessive and easily agglomerates upon polymerization, which prevents a uniform polymer layer from being produced. When the weight ratio is less than 0.1/99.9, in turn, a functional organic compound can not be fully reacted and bonded so that effects of UV absorbing performance, skin-chapping preventing performance, or the like may not sufficiently be obtained.

### (Third Modified Inorganic Fine Particles for Cosmetics)

Third modified inorganic fine particles according to the present invention are prepared by bonding a functional group of the polymer layer (II) of the second modified inorganic fine particles with one or more members selected from among dyes, UV absorbers and skin-chapping inhibitors as a functional organic compound(s).

Dyes used herein may be any organic dye as far as it is capable of bonding with the functional group of the polymer layer (II), and acid dyes having a functional group such as sulfonic acid sodium, carboxylic acid sodium, or the like are especially preferable in view of reactivity with the functional group. One or a mixture of two or more of these dyes may also be used. The quantity of dyes bonded with the functional group and fixed in the polymer layer (II) is preferably in the range from 1 to 20% by weight, and especially preferably, in the range from 5 to 15% by weight, to the weight of the inorganic fine particles forming the polymer layer (II). When the quantity of dyes is less than 1% by weight, coloring effect is low, while, when the quantity of dyes is more than 20% by weight, it is difficult to fix further more dyes.

UV absorbers used herein may be any UV absorber as far as it is capable of bonding with the functional group of the polymer layer (II), and preferably, it has a functional group such as a hydroxyl group, a sulfonic acid sodium group, a carboxylic acid group, or the like in view of reactivity with the functional group. After the functional group described above reacts and bonds with the polymer layer (II), however, the UV absorber is required to retain the UV absorbing performance therein, and, in this view, dihydroxy dimethoxybenzophenone disulfonic acid sodium is especially preferable. The quantity of UV absorbers fixed in the polymer layer (II) is preferably in the range from 1 to 20% by weight to the weight of the inorganic fine particles forming the polymer layer (II). When the quantity of UV absorbers is less than 1% by weight, UV blocking effect is low, while, when the quantity of UV absorbers is more than 20% by weight, it is difficult to fix further more UV absorbers.

Skin-chapping inhibitors used herein may also be selected in view of reactivity with the functional group as described above, and any skin-chapping inhibitors having a functional group such as a carboxylic acid group, an amino acid group, a hydroxyl group or the like is preferable. More specifically, multiple alcohols such as glycerin, mucopolysaccharides such as sodium hyaluronate, various amino acids such as water-soluble collagen, serine, or the like are listed for the possible use herein. In addition, anti-aging agents such as vitamins, whitening agents such as arbutin, organic compounds having infrared absorbing performance may be used similarly.

Further, some compounds included in the skin-chapping inhibitors, anti-aging agents, whitening agents, UV absorbers, or the like have relatively large molecular weight (having the molecular weight of approximately 1000 or more), in which case the compounds may be physically fixed entwining with the mesh of the polymer layer (II), in addition to the chemical bond.

The quantity of the skin-chapping inhibitors to be fixed is preferably in the range from 0.5 to 20% by weight, and especially preferably, in the range from 2 to 10% by weight, to the weight of the inorganic fine particles forming the polymer layer (II). When the quantity of skin-chapping inhibitors is less than 0.5% by weight, the effect is not sufficiently obtained, while, when the quantity of UV absorbers is more than 20% by weight, it is difficult to fix further more skin-chapping inhibitors.

Various methods can be employed as the method of forming the polymer layer (III) by fixing a functional organic compound in the polymer layer (II). Table 1 shows some examples of a combination of the functional group between the polymer layer (II) prepared by plasma graft-polymerization and the functional organic compound to be fixed.

**Table 1**

| Functional group of polymer layer (II) | Functional group of functional organic compound |
|---|---|
| Amido group | Sulfonic acid salt |
| idem | Carboxylic acid salt |
| idem | Glycidyl group |
| Glycidyl group | Amido group |
| idem | Hydroxyl group |
| idem | Carboxylic acid salt group |
| Carboxylic acid group | Hydroxyl group |
| idem | Amido group |
| idem | Amine group |
| Isocyanate group | Hydroxyl group |
| idem | Amine group |
| idem | Oxazoline compounds |
| idem | Sulfonic acid salt |
| idem | Ammonium salt |

In order to promote reaction between the functional groups described above, catalyst may be used therein or heat may be applied thereto. A preferable example is a method of forming the polymer layer (II) with a monomer having an amido bond such as acrylamide and fixing thereto a dye having a sulfonic acid sodium group. In the method, after treating the inorganic fine particles generating therein the polymer layer (II) with, for example, hydrochloric acid acidic solution, the inorganic fine particles are mixed in a dye solution to fix the dye in the polymer layer (II), so that the surface-modified inorganic fine particles according to the present invention can be obtained. In order to promote the fixing, heat treatment may be applied.

### (Cosmetics)

Cosmetics according to the present invention is preferably blended with the modified inorganic fine particles described above in the range from 1 to 90% by weight, and especially preferably, in the range from 3 to 60% by weight. When the blended quantity of modified inorganic fine particles is less than 1% by weight, blending effect can not be obtained, while, when the quantity thereof is more than 90% by weight, coloring, oily nature, or the like, which are properties sought for cosmetics in itself is lost.

Cosmetics according to the present invention includes at least any one of various ingredients blended in commonly used cosmetics, for example, oil contents such as higher aliphatic alcohols, higher fatty acids, ester oil, paraffin oil and wax; alcohols such as ethyl alcohol, propylene glycol, sorbitol, glycerin; moisturizing agents such as mucopolysaccharides, collagens, PCA salts and lactates; nonionic, cationic, anionic, amphoteric or other various surfactants; thickening agents such as gum arabic, xanthan gum, polyvinyl pyrrolidone, ethyl cellulose, carboxy methyl cellulose,' carboxy vinyl polymer, and metamorphic or unmetamorphic clay minerals; solvent such as ethyl acetate, acetone and toluene; inorganic pigments and dyes, organic pigments and dyes, BHT, oxidation inhibitors such as tocopherol, water, medical agents, UV absorbers, pH buffering agents, chelating agents, preservatives, fragrant materials, and the like. Further, at least one or more members selected from among inorganic bulking agents such as silica, talc, kaolin and mica, body colors, various organic resin, or the like may be included therein. Powdery ingredients thereof such as inorganic bulking agents, body colors, various organic resin, and various inorganic pigments and dyes may be surface-treated with silicon treatment, fluorine treatment, metallic soap treatment, or the like.

Cosmetics according to the present invention can be produced with conventional procedures, and may be used in various forms such as in the form of powder, cake, pencil, stick, liquid, cream, or the like, and more specifically, in the form of foundation, cream, emulsion, eye shadow, makeup base, nail enamel, eye liner, mascara, lipstick, facial mask, hair cosmetic, or the like.

### (Effect of the Invention)

The modified inorganic fine particles for cosmetics according to the present invention are formed on a surface thereof with a polymer layer prepared by plasma polymerization or plasma graft polymerization, so that the polymer layer is uniformly formed having firm bonds thereof. With this feature, the inorganic fine particles are excellent in harmlessness without disadvantageously attaching to or being absorbed into the skin.

Third modified inorganic fine particles for cosmetics have, according to the type of functional organic compounds, a significantly high chroma, a bright color tone, and color tone stability, are excellent in UV blocking performance and skin-chapping preventing performance (moisturizing performance), and provide water and oil repellency, so that the inorganic fine particles are advantageously used for cosmetics excellent in dispersing stability and long-lasting nature or the like after application thereof to the skin.

The present invention is described further specifically with reference to the examples shown below.

### (Example 1)

10 g of mica with the average particle size of 12 µm and thickness of 0.3 µm was put in a rotating container made of pyrex glass in a low-temperature plasma device for fine particles surface treatment, which was then evacuated to displace the inside thereof with argon gas and to be adjusted to have a pressure of 0.1 Torr. By applying high-frequency electric power to a copper pipe coil wound around an outer circumference of the container, plasma was generated in the rotating container with the induction system to irradiate plasma on a surface of the mica particles. Plasma irradiation conditions were as follows:

| | |
|---|---|
| Container rotating velocity | 24 rpm |
| Discharge gas | Ar |
| Gas flow rate | 3.3 × 10⁻² dm³/minute |
| Power | 200 W |
| Discharge time | 5 minutes |
| Pressure | 0.1 Torr |

The plasma-irradiated mica was transferred into a container with 350 ml of an isopropyl alcohol solution having acrylamide (1 mol/l) and glycidyl methacrylate (1 mol/l) without exposing to the atmosphere, and the mixture was heated to 70°C on an oil bath for reaction, so that the acrylamide and glycidyl methacrylate were graft-copolymerized to form the polymer layer (II) on a surface of the mica particles, which was then vacuum-dried at room temperature. The mica coated with the copolymers of acrylamide and glycidyl methacrylate was calcinated at a temperature of 700°C for 3 hours, and the weight percentage of the polymer layer (II) was then calculated with the ignition loss method to find the result to be 11.5%. 5g of the mica particles forming the polymer layer (II) was impregnated in a hydrochloric acid acidic solution adjusted to have pH 3 or below for 5 minutes, and was then washed with deionized water. The resultant mica was put in a 500 ml of 1% by weight aqueous solution with the red dye No. 227 (disodium salt of 8-amino-2-phenylazo-1-naphthol-3,6-disulfonic acid) . to continue stirring for 1 hour, and was then filtrated and dried. After that, the mica was applied to heat treatment at 200°C for 1 hour to obtain the modified inorganic fine particles (A) fixing the red dye No. 227 therein. The weight percent of the fixed red dye No. 227 to the obtained modified inorganic fine particles (A) was 12% by weight, which was calculated from the change in weight before and after fixing the red dye No. 227. When the modified inorganic fine particles (A) were suspended in water and agitated vigorously, the supernatant liquid thereof remained clear and colorless so that the red dye No. 227 was found to be firmly fixed with the polymer layer (II).

### (Example 2)

The modified inorganic fine particles (B) fixing dihydroxy dimethoxybenzophenone sulfonic acid sodium to the polymer layer (II) was obtained with the same method as that employed in Example 1 except the points that sericite, instead of mica, with the average particle size of 15 µm and thickness of 0.2 µm is used and that dihydroxy dimethoxybenzophenone sulfonic acid sodium, instead of the red dye No. 227, as a UV absorber was used. The sericite coated with copolymers of acrylamide and glycidyl methacrylate was calcinated at a temperature of 700°C for 3 hours, and the weight percentage of the polymer layer (II) was then calculated with the ignition loss method to find the result to be 13.2% by weight. Further, the weight percent of the dihydroxy dimethoxybenzophenone sulfonic acid sodium in the modified inorganic fine particles (B) was 7% by weight, which was calculated with the same method as that employed in Example 1.

The modified inorganic fine particles (B) were suspended in water to have a dispersing concentration of 10% by weight, were then agitated vigorously for 10 minutes, and were filtrateed to demonstrate that the concentration of dihydroxy dimethoxybenzophenone sulfonic acid sodium in the filtrate was not more than 5 ppm through measurement with the liquid chromatograph/mass spectrometer (GCMS).

The modified inorganic fine particles (B) were homogeneouslly dispersed by adding glycerin thereto to have a dispersing concentration of 1% by weight, and were subjected to the measurement of spectral transmittance at a wavelength of 290 to 700 nm using the quartz cell 1 mm thick to observe an absorption peak at 330 nm, which revealed that the fine particles (B) have UV absorbing performance due to dihydroxy dimethoxybenzophenone sulfonic acid sodium. The sericite as well as the sericite coated with copolymers of acrylamide and glycidyl methacrylate were also subjected to the measurement of spectral transmittance employing the same method as described above to observe no peak at 330 nm in either case.

### (Example 3)

10 g of spherical silica fine particles with the average particle size of 15 µm were put in a rotating container made of pyrex glass in a low-temperature plasma device for fine particles surface treatment, which was then evacuated to displace the inside thereof with argon gas and to be adjusted to have pressure of 0.1 Torr. By applying high-frequency electric power to a copper pipe coil wound around an outer circumference of the container, plasma was generated in the rotating container with the induction system to plasma-treat a surface of the spherical silica fine particles. Plasma treatment conditions were as follows:

| | |
|---|---|
| Container rotating velocity | 24 rpm |
| Discharge gas | Ar |
| Gas flow rate | 3.3 × 10⁻² dm³/minute |
| Power | 200 W |
| Discharge time | 5 minutes |
| Pressure | 0.1 Torr |

The treated spherical silica fine particles were transferred into a container with 750 ml of an isopropyl alcohol solution having glycidyl methacrylate (1 mol/l) without exposing to the atmosphere, and the mixture was heated to 70°C on an oil bath for reaction, so that the polymer layer (II) with glycidyl methacrylate was formed on a surface of the spherical silica fine particles, which was then vacuum-dried at room temperature. The spherical silica fine particles forming the polymer layer (II) with glycidyl methacrylate was calcinated at a temperature of 700°C for 3 hours, and the weight percentage of the polymer layer (II) was then calculated with the ignition loss method to find the result to be 8.2 % by weight. 5 g of the spherical silica fine particles forming the polymer layer (II) was added into a 80 ml of 5 % by weight aqueous liquid of serine, namely, a neutral amino acid, and was then dewatered using an evaporator, and the resultant solid was heated at 170°C for 1 hour. After that, the solid was washed with deionized water to wash away unreacted serine and was dried to obtain the modified inorganic fine particles (C) fixing therein serine as a moisturizing agent in the polymer layer (II). The weight percent of the serine in the modified inorganic fine particles (C) was 6% by weight. When the modified inorganic fine particles (C) were applied to the skin, smooth and dewy feel was obtained.

On the other hand, when spherical silica fine particles were applied to the skin, something foreign was felt thereon. When the spherical silica fine particles coated with glycidyl methacrylate polymers were applied to the skin, dewy feel was not obtained, but powdery and dry texture was felt.

### (Example 4)

10 g of rice-grain formed red iron oxide particles with the average. particle size of 0.3 µm were put in a rotating container made of pyrex glass in a low-temperature plasma device for fine particles surface treatment, which was adjusted to have a flow rate of hexafluorobenzene with a pressure of 0.1 Torr and was gas-displaced. By applying high-frequency electric power to a copper pipe coil wound around an outer circumference of the container, plasma was generated in the rotating container with the induction system to plasma-treat a surface of the red iron oxide particles under the following conditions, so that the modified inorganic fine particles (D) forming the polymer layer (I) through plasma polymerization of hexafluorobenzene were obtained:

| | |
|---|---|
| Container rotating velocity | 24 rpm |
| Power | 50 W |
| Discharge time | 30 minutes |
| Pressure | 0.05 Torr |

The weight percent of the polymer layer (I) in the modified inorganic fine particles (D) calculated by the thermo-weight analysis was 6.8 % by weight.

In order to evaluate water repellency of the modified inorganic fine particles (D), the particles were shaped to be flat as sheet glass, which was then used as a sample, and the contact angle thereof was measured to be 128° using a contact angle meter with the water droplet method.

The modified inorganic fine particles (E) and the modified inorganic fine particles (F) each plasma polymerized with hexafluorobenzene were obtained with the same method as described above using rice-grain formed yellow iron oxide particles with the average particle size of 0.4 µm and non-spherical black iron oxide particles with the average particle size of 0.25 µm respectively. The contact angle thereof was 125° and 135° respectively, and both of the particles showed high water repellency.

On the other hand, rice-grain formed red iron oxide particles, rice-grain formed yellow iron oxide particles, and non-spherical black iron oxide particles were subjected to the measurement of contact angle without plasma polymerizing hexafluorobenzene, however, a droplet of each particles was absorbed into each sample in a moment of the dropping, so that measurement of the contact angle of each particles was not possible. Namely, the contact angle of each particles was 0°, unnecessary to measurement, and respective particles showed high hydrophilic nature.

### (Example 5)

The modified inorganic fine particles (G) forming the plasma polymer layer (I) with ethyl trimethylsilane were obtained with the same method as that employed in Example 4 except the points that non-spherical titanium oxide particles, instead of rice-grain formed red iron oxide particles, with the average particle size of 0.3 µm were used, and that ethyl trimethylsilane, instead of hexafluorobenzene, was used. The contact angle of the particles was measured to be 131 °, showing high water repellency thereof. On the other hand, the contact angle of non-spherical titanium oxide particles was 0°, showing high hydrophilic nature thereof.

### (Example 6)

The following Material A to Material C were mixed at a blend ratio (% by weight) described below corresponding to each ingredient to prepare pale pink emulsion.

Both Material A and Material B were heated at 80°C for dissolution, and Material B was added little by little to Material A while stirring to emulsify the mixture. The mixture was then cooled while stirring, and when the temperature thereof dropped to 40°C, Material C was added thereto. After homogenization, stirring was stopped and the mixture was left untouched to prepare pale pink emulsion.

| | | |
|---|---|---|
| A. | Polyoxyethylene sorbitan monostearate | 1.0 |
| | Tetraoleic acid polyoxyethylene sorbitol | 1.5 |
| | Glyceryl monostearate | 1.5 |
| | Stearic acid | 0.5 |
| | Biphenyl alcohol | 1.0 |
| | Cetyl Palmitate | 0.5 |
| | Squalene | 5.0 |
| | 2-ethyl hexanoic acid cetyl | 4.0 |
| | Methyl polysiloxane | 0.5 |
| | Preservative | proper quantity |
| B. | 1,3-butylene glycol | 10.0 |
| | Xanthan gum | 0.1 |
| | Purified water | 69.4 |
| C. | Modified inorganic fine particles (A) | 5.0 |

When this emulsion was applied to the skin, pale pink finishing having natural gloss was obtained. Although organic dyes produced with the conventional technology have had a safety problem including permeability to the skin, the fine particles fixed in mica according to the present invention provide a wider variety of cosmetics to be blended therewith.

### (Example 7)

Material A and Material B as follows were mixed at a blend ratio (% by weight) described below corresponding to each ingredient to prepare powder foundation.

Material A was mixed together homogeneously, and Material B was well stirred and mixed together while heating to 70°C. Material A was added to Material B to mix homogeneously, and the mixture was then pulverized and compression-molded to prepare powder foundation.

| | | |
|---|---|---|
| A. | Titanium oxide | 10.7 |
| | Red iron oxide | 0.55 |
| | Yellow iron oxide | 2.5 |
| | Black iron oxide | 0.15 |
| | Talc | 22.0 |
| | Mica | 22.1 |
| | Modified inorganic fine particles (B) | 28.0 |
| B. | Silicone oil | 9.0 |
| | Squalene | 3.2 |
| | Ester oil | 1.6 |
| | Sorbitan sesquiolate | 0.2 |
| | Fragrant materials | proper quantity |
| | Preservative | proper quantity |

When the sun protection factor (SPF) of this powder foundation was measured with the SPF analyzer (Optometrics USA, Inc. SPF-290), the value thereof was 14.2, showing an excellent UV blocking effect.

### (Comparative Example 1)

Powder foundation was prepared with the same method as that employed in Example 7 except the point that sericite used in Example 2, instead of the modified inorganic fine particles (B), was used. The SPF of this powder foundation was measured with the same method as that employed in Example 7 to find the value thereof to be 4.5, not showing a sufficient UV blocking effect.

### (Example 8)

Material A and Material B as follows were mixed at a blend ratio (% by weight) described below corresponding to each ingredient to prepare powder foundation.

Material A was mixed together homogeneously, and Material B was well stirred and mixed while heating to 70°C. Material A was added to Material B to mix homogeneously, and the mixture was then pulverized and compression-molded to prepare powder foundation.

| | | |
|---|---|---|
| A. | Modified inorganic fine particles (G) | 10.7 |
| | Modified inorganic fine particles (D) | 0.55 |
| | Modified inorganic fine particles (E) | 2.5 |
| | Modified inorganic fine particles (F) | 0.15 |
| | Talc (2% silicone treatment) | 22.0 |
| | Mica (2% silicone treatment) | 22.1 |
| | Sericite (2% silicone treatment) | 28.0 |
| B. | Silicone oil | 9.0 |
| | Squalene | 3.2 |
| | Ester oil | 1.6 |
| | Sorbitan sesquiolate | 0.2 |
| | Fragrant materials | proper quantity |
| | Preservative | proper quantity |

This powder foundation was applied to the face of a subject and was then untouched while the subject passed 3 hours in a room controlling the temperature at 26°C and the relative humidity at 78%. Change in color tone thereof over time was then examined, showing little change in tone color.

### (Comparative Example 2)

Powder foundation was prepared with the same method as that employed in Example 8 except the points that titanium oxide used in Example 5, instead of the modified inorganic fine particles (G), was used and that rice-grain formed red iron oxide particles, rice-grain formed yellow iron oxide particles and non-spherical black iron oxide particles used in Example 4, instead of the modified inorganic fine particles (D), the modified inorganic fine particles (E) and the modified inorganic fine particles (F), were used respectively. Change over time in color tone of this powder foundation was examined with the same method as that employed in Example 8, showing a change in tone color to be darker after 1 hour and to be considerably dull after 3 hours.

## Claims

1. Modified inorganic fine particles for cosmetics having on a surface thereof, a polymer layer (I) prepared by plasma-polymerizing one or more monomers selected from the group consisting of fluorinated monomers, silicone-based monomers, aromatic monomers and alicyclic monomers.

2. The modified inorganic fine particles for cosmetics according to claim 1, wherein the weight ratio of said polymer layer (I) to said inorganic fine particles is in the range from 0.1/99.9 to 30/70.

3. Modified inorganic fine particles for cosmetics having on a surface thereof, a polymer layer (II) prepared by plasma graft-polymerizing a monomer having a functional group.

4. The modified inorganic fine particles for cosmetics according to claim 3, wherein the weight ratio of said polymer layer (II) to said inorganic fine particles is in the range from 0.1/99.9 to 30/70.

5. The modified inorganic fine particles for cosmetics according to claim 3 or 4, wherein said modified inorganic fine particles for cosmetics have a polymer layer (III) bonding a functional organic compound(s) with said functional group.

6. The modified inorganic fine particles for cosmetics according to claim 5, wherein said functional organic compound(s) is one or more members selected from the group consisting of dyes, UV absorbers and skin-chapping inhibitors.

7. The modified inorganic fine particles for cosmetics according to claim 5 or 6, wherein the bonding quantity of said functional organic compound(s) to the weight of the inorganic fine particles forming said polymer layer (II) is in the range from 1 to 20% by weight.

8. Cosmetics blending 1 to 90% by weight of the modified inorganic fine particles for cosmetics according to any of claims 1 to 7.
